(19) Europäisches Patentamt
European Patent Office
Office européen des brevets

(11) Publication number : **0 587 374 A2**

# (12) EUROPEAN PATENT APPLICATION

(21) Application number : **93306936.1**

(22) Date of filing : **02.09.93**

(51) Int. Cl.$^5$ : **C07J 31/00,** A61K 31/575, C07J 71/00, A61K 31/58

(30) Priority : **02.09.92 ZA 926649**

(43) Date of publication of application : **16.03.94 Bulletin 94/11**

(84) Designated Contracting States : **AT BE CH DE DK ES FR GB GR IE IT LI LU MC NL PT SE**

(71) Applicant : **VIROSTAT (NA) NV**
**C E B Hellmundweg 11**
**Kralendijk, Bonaire (NA)**

(72) Inventor : **Kreger, Petrus Buys**
**11 Comrie Road**
**Camps Bay Cape Province (ZA)**

(74) Representative : **Ellis-Jones, Patrick George Armine**
**J.A. KEMP & CO. 14 South Square Gray's Inn**
**London WC1R 5LX (GB)**

## (54) Sulfated sterol glycosides and their use against viral infections.

(57) The invention discloses a chemical compound which is a sulphated steryl glycoside or a sulphated spiroketal steroid glycoside. The compound has a hydrophobic portion selected from sterol aglycone groups and spiroketal steroid aglycone groups each having a 3-oxy-glycoside linkage, a saccharide unit coupled to the aglycone group via its 3-oxy-glycoside linkage and at least one sulphate group which is a sulphated ester or an hydroxyl group of the saccharide unit. The invention extends to a compound, substance or composition comprising the chemical compound, for use in the treatment or prophylaxis of a viral infection in a human or animal subject, to a compound, substance or composition comprising the compound for use in the preparation of a medicament and to a medicine or medicament.

EP 0 587 374 A2

THIS INVENTION relates to a chemical compound; to a method for the preparation of said chemical compound; to a compound, substance or composition, comprising said chemical compound, for use in the treatment or prophylaxis of a viral infection in human or animal subject; to a substance or composition, comprising said chemical compound, for use in the preparation of a composition, medicine or medicament for treating or for use in prophylaxis of a viral infection in a human or animal subject; to use of a compound, substance or composition in the preparation of a composition, medicine or medicament for treating or preventing a viral infection in a human or animal subject; to a medicine or medicament for use in the treatment or prophylaxis of a viral infection in human or animal subject; to a compound, substance or composition for use as a medicine or medicament; to a compound, substance or composition for use as an active therapeutic or prophylactic agent; and to a method of treatment, in a human or animal subject, of a viral infection.

According to the invention there is provided a chemical compound which comprises a sulphated glycoside selected from sulphated steryl glycosides and sulphated spiroketyl steroid glycosides, the sulphated glycoside having:

a hydrophobic portion selected from sterol aglycone groups having steryl groups and spiroketal steroid aglycone groups having spiroketyl steroid groups, each said aglycone group having a 3-oxy-glycoside linkage;

at least one saccharide unit coupled to said aglycone group via the 3-oxy-glycoside linkage of said aglycone group; and

at least one sulphate group which is a sulphated ester of a hydroxyl group which forms part of said saccharide unit.

In other words, according to this aspect of the invention there is provided a chemical compound which comprises a sulphated steryl glycoside or sulphated spiroketyl steroid glycoside having:

a hydrophobic portion comprising a sterol aglycone group or a spiroketal steroid aglycone group;

at least one saccharide unit coupled to the steryl group or to the spiroketyl steroid group via the steryl 3-oxy-glycoside ether linkage or the spiroketyl steroid 3-oxy-glycoside ether linkage; and

at least one sulphate group which is a sulphated ester of a hydroxyl group forming part of a said saccharide unit.

When the compound is a steryl glycoside the aglycone moiety is preferably cholesterol or β-sitosterol, although it may be a related phytosterol such as campesterol, stigmasterol or spinasterol. Thus, the compound may be a sulphated steryl glycoside whose aglycone group has an aglycone moiety which is a phytosterol selected from cholesterol, β-sitosterol, campesterol, stigmasterol and spinasterol. When the compound is a spiroketal steroid glycoside the aglycone moiety may be diosgenin, tigogenin or hecogenin. Accordingly, the compound may be a sulphated spiroketyl steroid glycoside whose aglycone group has an aglycone moiety selected from diosgenin, tigogenin and hecogenin. Each saccharide unit is, as mentioned above, coupled to the sterol aglycone group or spiroketal steroid aglycone group via the aglycone 3-oxy glycoside ether linkage. Each saccharide unit is preferably a monosaccharide unit derived from a pentose or a hexose. The monosaccharide unit is, more preferably, a glucoside unit, but saccharide units comprising other monosaccharides, disaccharides or higher saccharides can also in principle be employed. In other words, the compound may be one in which each saccharide unit is selected from monosaccharide units derived respectively from pentoses and hexoses, and monosaccharide units which are glucoside units. The compounds of the present invention include all the congeners of the above aglycones and their glycosides.

In the case of saccharide units comprising glucosides, mono-, di-, tri- and tetra-sulphates thereof are possible, the more highly sulphated glucosides being preferred. The sulphated glucosides are thus preferably tri- and tetra-sulphates. The compound may accordingly be one in which each saccharide unit is a glucoside unit which is sulphated and is selected from the mono-, di-, tri- and tetra- sulphates of glucoside units.

The compound may be in acid form, or it may be in the form of a salt whereby each sulphate anion may be associated with a suitable counterion such as a sodium cation, although ammonium, potassium, magnesium and calcium cations as well as various amine salts may instead be used. Thus, the compound may be in the form of a salt having at least one sulphate anion, each sulphate anion being associated with a counterion selected from cations consisting of sodium-, ammonium-, substituted ammonium, potassium-, magnesium- and calcium cations.

Preferred compounds according to the invention are:

β-sitosteryl β-D-glucoside tetra-sulphate;
cholesteryl β-D-glucoside tetra-sulphate;
campesteryl β-D-glucoside tetra-sulphate;
stigmasteryl β-D-glucoside tetra-sulphate;
spinasteryl β-D-glucoside tetra-sulphate;
diosgenyl β-D-glucoside tetra-sulphate;
tigogenyl β-D-glucoside tetra-sulphate;

hecogenyl β-D-glucoside tetra-sulphate; and
more preferred compounds are:
β-sitosteryl β-D-glucoside tetra-sulphate;
cholesteryl β-D-glucoside tetra-sulphate; and
diosgenyl β-D-glucoside tetra-sulphate,
being in the form of the sodium salt thereof.

According to another aspect of the invention there is provided a method for the preparation of a chemical compound as described above, the method comprising the step of sulphating a steryl glycoside.

While it is in principle possible to employ any suitable sulphating reagent such as a sulphur trioxide adduct of a Lewis base as a sulphate donor for the sulphation, chlorosulphonic acid in pyridine has successfully been employed as a sulphate donor. Thus, the sulphating step may be carried out using a sulphating agent selected from sulphur trioxide adducts of Lewis bases and chlorosulphonic acid in pyridine. Preferably the reaction is carried out in the absence of water. Thus the reaction may be carried out with the steryl glycoside or spiroketyl steroid glycoside dissolved in a solution of chlorosulphonic acid in pyridine. The reaction may be carried out at 95-100°C over a period of ½ - 2 hours, e.g. about 1 hour. In particular, thus, the sulphating step may be carried out in the absence of water, with the steryl glycoside dissolved in an organic solvent, at a temperature of 95-100°C and over a period of ½ - 2 hours.

The reaction mixture may, after completion of the reaction, be extracted with aqueous sodium hydroxide and the product purified by a series of selective precipitation steps utilising methanol or other suitable precipitant.

The invention extends also to a chemical compound which comprises a sulphated glycoside as described above, when prepared by a method described above.

The invention also extends to a chemical compound which comprises a sulphated glycoside as described above, the composition being in the form of a sterile injectable liquid in which the compound is dispersed, e.g. by being dissolved as a solute in an aqueous solvent; and the liquid is preferably a saline solution, the compound being dispersed therein by being dissolved as a solute therein at a concentration of 0.001-20 g/ℓ, preferably 1-20 g/ℓ and more preferably 1-10 g/ℓ.

According to another aspect of the invention there is provided a compound, substance or composition for use in the treatment or prophylaxis of a viral infection in a human or animal subject, said compound, substance or composition comprising a sulphated glycoside as described above.

According to a further aspect of the invention there is provided a compound, substance or composition for use in the preparation of a composition, medicine or medicament for treating or for the prophylaxis of a viral infection in human or animal subject, said compound, substance or composition comprising a sulphated glycoside as described above.

According to a still further aspect of the invention there is provided the use of a compound, substance or composition in the preparation of a composition, medicine or medicament for treating or preventing a viral infection in a human or animal subject, said compound, substance or composition comprising a sulphated glycoside as described above.

According to a yet further aspect of the invention there is provided a medicine or medicament for use in the treatment or prophylaxis of a viral infection in human or animal subject, the medicine or medicament comprising a sulphated glycoside as described above.

The medicine or medicament may be a sterile injectable liquid in which the compound comprising the sulphated glycoside is dispersed. In particular, the medicine or medicament may be an injectable solution comprising an aqueous, e.g. a saline, solution having said sulphated steryl glycoside or sulphated spiroketyl glycoside salts dispersed therein, e.g. by being dissolved therein. In other words, the compound comprising the sulphated glycoside may be dissolved in the liquid to form a solution, the solution comprising a sterile saline solution.

The concentration of the sulphated steryl glycoside or sulphated spiroketyl steroid glycoside in the saline solution may be 0,001 - 20 g/ℓ and may particularly be between 1 - 20 g/ℓ, e.g. 1-10 g/ℓ. Thus, the concentration of the compound comprising the sulphated glycoside in the injectable liquid may be 1-20 g/ℓ, preferably 1-10 g/ℓ.

The invention extends to a compound, substance or composition for use as a medicine or medicament, the compound, substance or composition comprising a compound which is a sulphated glycoside as described above.

The invention extends further to a compound, substance or composition for use as an active therapeutic or prophylactic agent, the compound, substance or composition comprising a compound which is a sulphated glycoside as described above.

The invention makes possible a method of treating a viral infection in a human or animal subject, which method comprises administering, to the subject, a chemical compound which comprises a sulphated glycoside

selected from sulphated steryl glycosides and sulphated spiroketyl steroid glycosides, the sulphated glycoside having:

a hydrophobic portion selected from sterol aglycone groups having steryl groups and spiroketal steroid aglycone groups having spiroketyl steroid groups, each said aglycone group having a 3-oxy-glycoside linkage;

at least one saccharide unit coupled to said aglycone group via the 3-oxy-glycoside linkage of said aglycone group; and

at least one sulphate group which is a sulphated ester of a hydroxyl group which forms part of said saccharide unit.

The sulphated glycoside may be a sulphated steryl glycoside or sulphated spiroketyl steroid glycoside as described in more detail above and may be administered as a medicine or medicament as described above, e.g. by parenteral administration, as described in more detail hereunder.

The anti-viral activity of the compounds of the invention is expected to extend to a variety of viral types. For example, in-vitro trials have revealed that β-sitosteryl β-D-glucoside tetra-sulphate, and analogous sulphated glycosides of cholesterol and diosgenin, all have anti-HIV-1 (anti-Human Immunodeficiency Virus Type 1) activity. The inhibition of viral activity in these trials was based on the inhibition of production of the HIV core protein, P24, together with inhibition of the viral cytopathic effect on the lymphocyte host cells (set forth in more detail in Tables 1, 2 and 3 hereunder).

β-Sitosteryl β-D-glucoside tetra-sulphate (BSSG-$(SO_4^-)_4$) was also evaluated in-vitro for anti-FIPV (anti-Feline Infectious Peritonitis Virus) activity. The substance was tested at 5, 12.5, 25 and 50 μg/mℓ and at three viral dilutions namely $10^2$-, $10^3$-and $10^4$-fold dilutions where the original titre results in the production of between 50 and 100 mg of P24 viral core protein/mℓ of tissue culture medium and target cells after 4 days of incubation. The viral cytopathic effect was completely inhibited at all these test conditions, whereas the cytopathic effect was complete in each control where the virus was administered without the drug. The drug alone had no cytopathic effect.

Furthermore, BSSG-$(SO_4^-)_4$ was evaluated in vivo for anti-FIV (anti-Feline Immunodeficiency Virus) activity. The substance was tested at a dosage rate of 5 mg/kg body mass on a cat infected with feline immunodeficiency virus, administered by subcutaneous injection at weekly intervals over a period of 13 weeks. Within 2 weeks serum antibody titer decreased and a further decrease was evident after 13 weeks, indicative of decreases in antigen load and viral load. Over the test period increases in cell counts were observed for white blood cells, lymphocytes, $CD3^+$, $CD4^+$, $CD8^+$ and T-helper cells, indicative of successful treatment, at least over the 13 week test period.

The cytopathic effect of Feline Herpes virus in further tests was delayed by a day in the presence of BSSG-$(SO_4^-)_4$ at a concentration of 50 μg/mℓ.

The steryl glycoside sulphates and spiroketyl steroid glycoside sulphates of the present invention are highly soluble in aqueous media. Aqueous solutions of these substances may therefore be formulated according to standard practice for purposes of parenteral administration, e.g. by intravenous, subcutaneous and intramuscular injection. The substances may in principle however, instead, be administered orally or topically. Topical application of steryl glucoside sulphates in a cream or ointment base may, for example, be used in the treatment of dermal viral infections such as herpes simplex. Formulations of the substance into a vaginal cream or pessary may be used in gynaecological conditions such as herpes infections. Steryl glucoside sulphates or spiroketyl steroid glucoside sulphates may be used as anti-viral substances for both human and veterinary use.

The invention will now be described by way of example with reference to tests conducted to demonstrate in vitro anti-viral activity against HIV-1, in vivo toxicological and pharmacokinetic studies, in vitro cytotoxicity studies, and Examples of the preparation of the glycoside sulphates.

These tests are illustrated by the accompanying drawings, in which:

Figure 1 is a thin layer chromatogram of various steryl and spiroketyl steroid congeners as used in the tests; and

Figure 2 is a histogram of blood concentration levels of the BSSG- $(SO_4^-)_4$ against time in a chacma baboon.

The following abbreviations will be used:

BSS = β-Sitosterol.
BSS-$SO_4^-$ = β-Sitosteryl sulphate.
BSSG = β-Sitosteryl β-D-glucoside.
BSSG-$(SO_4^-)_4$ = β-Sitosteryl β-D-glucoside tetra-sulphate.
CHO = Cholesterol.
CHO-$SO_4^-$ = Cholesteryl sulphate.
CHOG = Cholesteryl β-D-glucoside.
CHOG-$(SO_4^-)_4$ = Cholesteryl β-D-glucoside tetra-sulphate.

D = Diosgenin.
$D\text{-}SO_4^-$ = Diosgenyl sulphate.
DG = Diosgenyl β-D-glucoside.
$DG\text{-}(SO_4^-)_4$ = Diosgenyl β-D-glucoside tetra-sulphate.

It is to be noted that, in use, as described herein, the steryl sulphates of the present invention will typically be in the form of suitable salts thereof (e.g. $NH_4^+$, $K^+$, $Mg^{2+}$, $Ca^{2+}$ or the like salts), preferably the Na+ salts.

*In-Vitro* Anti-HIV-1 Tests

Peripheral human lymphocytes (PBL's) obtained from different donors at a blood bank were pooled and used for the propagation of HIV-1 in vitro. The PBL's were polyclonally activated with phytohemagglutinin (PHA) and subjected to quality controls whereby their viability was tested with Trypan Blue exclusion; and the expression of activation antigens, $CD4^+$ and $CD25^+$, was measured by immunofluorescence. Prior to adding the test substance, the activated PBL's were exposed to a standardized titre of HIV-1 virus and, after incubation for 60 minutes, the cells were washed twice and transferred into microtitre plates at a concentration of $2 \times 10^6$ cells/well. The medium contained interleukin 2 (IL-2) and, in control wells, the virus produced between 50 and 100 ng/mℓ of core antigen P24 protein on day 4. The test substance was added to these cultures either in water or aqueous dimethyl sulphoxide (DMSO) solutions, depending on its solubility characteristics. The final DMSO concentration was always 1 % by mass. The effect of each test substance was compared to that of zidovudine i.e. 3'-azido-3'-deoxythymidine (AZT) at concentrations of 100, 10, 1 and 0.1 ng/mℓ.

HIV-1 virus replication was measured by the production of core antigen P24 protein and by the production of HIV-1 ribonucleic acid (RNA) on days 2, 3 and 4, or on days 3, 4, 5 and 6. Core antigen protein P24 was measured employing 'Sandwich ELISA' technology using, inter alia, a monoclonal antibody against P24. The HIV-1 RNA was measured using a specifically labelled 2-deoxyribonucleic acid (DNA) probe against HIV-1 RNA and the process of hybridization.

Results were measured as percentage inhibition of HIV-1 virus replication relative to control values. Possible toxic effects of the test substance were measured by viability testing using Trypan Blue or a proliferation assay using 3-[4,5-dimethylthiazol-2-yl)2,5-diphenyl-2H-tetrazolium bromide (MMT) on day 4 or day 6 after initiation of the experiment. Instead, the corresponding chloride can be used.

It is to be noted that an aqueous DMSO solution at 1 % by mass concentration does not inhibit HIV replication.

The results of these tests are set out in Tables 1, 2 and 3.

Table 1

| A Comparison of the In Vitro Anti-HIV-1 Effect of Various Sulphated Molecules. | | | | |
|---|---|---|---|---|
| TEST CODE REFERENCE | TEST SUBSTANCE | TEST CONCENTRATION (µg/mℓ) | DURATION OF TEST (DAYS) | % INHIBITION OF P24 PROTEIN PRODUCTION |
| ZZ 1A | BSSG-$(SO_4^-)_4$ | 50 | 6 | 100 |
| ZZ 1A | BSSG-$(SO_4^-)_4$ | 25 | 6 | 91 |
| ZZ 2A | n-OCTYL GLUCOSIDE TETRA-SULPHATE | 50 | 6 | 0 |
| ZZ 2A | n-OCTYL GLUCOSIDE TETRA-SULPHATE | 25 | 6 | 0 |
| ZZ 3A | ACCUMINOSIDE POLY-SULPHATE | 50 | 6 | 38 |
| ZZ 3A | ACCUMINOSIDE POLY-SULPHATE | 25 | 6 | 10 |
| ZZ 5A | DG-$(SO_4^-)_4$ | 50 | 6 | 100 |
| ZZ 5A | DG-$(SO_4^-)_4$ | 25 | 6 | 60 |
| ZZ 6A | SODIUM n-DODECYL SULPHATE | 50 | 6 | 45 |
| ZZ 6A | SODIUM n-DODECYL SULPHATE | 25 | 6 | 0 |

Accuminoside is a geraniol apiofuranoside from Hypoxis acuminata (Bredenkamp M.W., Drewes S.E. and Wenteler G.L., Phytochemistry, Vol. 28, 263 - 265 (1989).

BSSG-$(SO_4^-)_4$ (ZZ 1A) and DG-$(SO_4^-)_4$ (ZZ 5A) showed marked anti-HIV-1 effects compared with other sulphated substances whether they were sulphates of a glycoside (ZZ 2A and ZZ 3A) or sulphates of molecules that did not contain a glycoside (ZZ 6A).

Table 2

| The In Vitro Anti-HIV-1 Effects of β-Sitosterol and Cholesterol, their β-D-glucosides and their β-D-glucoside Tetra-sulphates. | | | | |
|---|---|---|---|---|
| TEST CODE REFERENCE | TEST SUBSTANCE | TEST CONCENTRATION (μg/mℓ) | DURATION OF TEST (days) | % INHIBITION OF P24 PROTEIN PRODUCTION |
| LL 1 | BSS-$SO_4^-$ | 10 | 6 | 0 |
| LL 2 | BSS | 10 | 6 | 0 |
| LL 3 | BSSG-$(SO_4^-)_4$ | 10 | 6 | 55.0 |
| LL 4 | BSSG | 10 | 6 | 0 |
| LL 5 | CHO | 10 | 6 | 3 |
| LL 6 | CHO-$SO_4^-$ | 10 | 6 | 0 |
| | | | | |
| 1/92-4 | BSSG-$(SO_4^-)_4$ | 100 | 4 | 99 |
| 1/92-5 | BSSG-$(SO_4^-)_4$ | 50 | 4 | 83 |
| 1/92-6 | BSSG-$(SO_4^-)_4$ | 25 | 4 | 61 |
| 1/92-9 | CHOG-$(SO_4^-)_4$ | 100 | 4 | 86 |
| 1/92-10 | CHOG-$(SO_4^-)_4$ | 50 | 4 | 49 |
| 1/92-11 | CHOG-$(SO_4^-)_4$ | 25 | 4 | 18 |
| 1/92-12 | CHOG-$(SO_4^-)_4$ | 12.5 | 4 | 19 |

The percentage inhibition of P24 protein synthesis was calculated with reference to the amount of P24 protein produced by the viral infected host cells without the presence of the test substance. Test samples of the sterol aglycones and their sulphates exhibited no significant inhibition of P24 protein production at 10 μg/mℓ (LL1, LL2, LL4, LL5, LL6) whereas BSSG-$(SO_4^-)_4$ inhibited P24 protein production by 55% (LL3).

The anti-HIV-1 effects of β-sitosteryl and cholesteryl β-D-glucoside tetra-sulphates were further illustrated at various test concentrations in tests 1/92-4 to 1/92-12. The inhibition of viral proliferation was shown to be dependent on the concentrations of the test substances in these tests.

Table 3

| Determination of the In Vitro Anti-HIV-1 Potency of β-sitosteryl β-D-glucoside tetra-sulphate (BSSG-$(SO_4^-)_4$. | | | | |
|---|---|---|---|---|
| TEST CODE REFERENCE | TEST SUBSTANCE | TEST CONCENTRATION (μg/mℓ) | DURATION OF TEST (Days) | % INHIBITION OF P24 PROTEIN PRODUCTION |
| 17/6 | BSSG-$(SO_4^-)_4$ | 50 | 4 | 96 |
| 17/12 | BSSG-$(SO_4^-)_4$ | 25 | 4 | 88 |
| 17/13 | BSSG-$(SO_4^-)_4$ | 12.5 | 4 | 65 |
| 17/14 | BSSG-$(SO_4^-)_4$ | 3.125 | 4 | 27 |
| 17/15 | BSSG-$(SO_4^-)_4$ | 0.78 | 4 | 4 |

By the extrapolation of the data in Table 3, BSSG-$(SO_4^-)_4$ was found to inhibit P24 protein synthesis by 50% at a concentration of 9 μg/mℓ. This value may vary from test to test depending on the particular lymphocyte batch used and/or with the batch titre of HIV-1 used.

AZT at a concentration of 10 ng/mℓ in this test was sufficient to produce 100% inhibition of P24 protein production.

From the data of Tables 1, 2 and 3 the following can be concluded:

β-sitosterol and cholesterol, their sulphates and their β-D-glucosides do not have anti-HIV-1 properties in terms of P24 protein production. Surprisingly, these sterols exhibit anti-HIV-1 activity only when presented as the sulphates of their glucosides. The spiroketyl steroid, diosgenin, also has anti-HIV-1 potential as a β-D-glucoside sulphate, as seen from Table 1.

Further tests indicate that the tetra-sulphated glucosides are more potent as anti-HIV substances than 2mixtures of the mono-, di-, tri- and tetra-sulphated glucosides of these aglycones.

From a number of tests it has been established that at 50 μg/mℓ BSSG-$(SO_4^-)_4$ gives consistent inhibition (i.e. 80 - 100%) of P24 protein synthesis.

The purity and identity of the compounds used in these in vitro anti-viral tests were monitored by thin layer chromatography (TLC) as illustrated in Figure 1, which as mentioned above, is a thin layer chromatogram of certain sterol and spiroketal steroids and their derivatives as used in the above in vitro anti-HIV-1 tests. In Figured 1 the TLC parameters are:

TLC Plate - Silica Gel G (Merck 5715) activated for 60 minutes at 90°C;
Developing Solvent Chloroform/ethanol/water in a ratio of 33:50:17 by volume;
Spray Reagent - Anisaldehyde/sulphuric acid.

In Figure 1, A-D refer to the following:

A - Rf Aglycones
B - Rf Glucosides
C - Rf Aglycone-$SO_4^-$
D - Rf Glucoside-$(SO_4^-)_4$

and the spot numbers correspond to the compounds and Rf values as follows:

| Spot Number | Compound Name | Rf Value |
|---|---|---|
| 1 | BSS | 0.99 |
| 2 | BSS-$SO_4^-$ | 0.81 |
| 3 | BSSG | 0.91 |
| 4 | BSSG-$(SO_4^-)_4$ | 0.53 |
| 5 | CHO | 0.98 |
| 6 | CHO-$SO_4^-$ | 0.83 |
| 7 | CHOG | 0.91 |
| 8 | CHOG-$(SO_4^-)_4$ | 0.52 |
| 9 | D | 0.98 |
| 10 | D-$SO_4^-$ | 0.80 |
| 11 | DG | 0.91 |
| 12 | DG-$(SO_4^-)_4$ | 0.51 |

## *In Vivo* Studies: Toxological Evaluation and Pharmacokinetics

The objective of the in vivo studies was to sustain anti-HIV-1 levels of BSSG-$(SO_4^-)_4$ in a primate and to evaluate the toxicological response. The potential for the use of this substance in man as an anti-viral (for example anti-HIV-1) therapeutic agent could thus be established.

The Chacma baboon was selected as the test animal and the evaluation was conducted in two stages.

Stage 1 Test

The Pharmacokinetics of BSSG-$(SO_4^-)_4$ in the Chacma Baboon

The purpose of the experiment was to study the pharmacokinetics of BSSG-$(SO_4^-)_4$ after intravenous administration thereof. Two test animals were used. Anaesthesia was induced in the test animals by the intramuscular administration of ketamine (5 mg/kg). Anaesthesia was maintained with halothane. An intravenous infusion of isotonic saline solution was administered to maintain a positive fluid balance of 8 mℓ/kg body mass. $^{35}$S-Isotope-labelled BSSG-$(SO_4^-)_4$ sodium salt in saline solution was infused intravenously using a mechanical infusion device (Perfusor Secura Braun, Melsungen). In the first baboon 750 mg was administered at a rate of 25 mg/min (1.85 mg/kg/min) for 30 minutes (total dose 55.6 mg/kg). The second baboon received 120 mg during 15 minutes (0.476 mg/kg/min, total dose 7.14 mg/kg). Twenty-five blood samples (1.5 mℓ each) were drawn from each test animal at intervals appropriate to the pharmacokinetic modelling. The plasma concentrations of BSSG-$(SO_4^-)_4$ were determined in the samples by interpolation from their radioactivity. The data were best described by a function containing two exponential terms from which an open two-compartment model was derived. The pharmacokinetic parameters as derived are presented in Table 4.

Table 4

| A Summary of the Pharmacokinetic Parameters Derived from the Intravenous Administration of BSSG-$(SO_4^-)_4$ to the Chacma Baboon. | | |
|---|---|---|
| | High Dose (750 mg) | Low Dose (120 mg) |
| Peak plasma concentration | 364 µg/mℓ | 102 µg/mℓ |
| Elimination half-life | 22 hours | 12 hours |
| Volume of distribution at steady state | 144 mg/kg | 64 mg/kg |
| Total boby clearance | 0.21 mg/min/kg | 0.068 mg/min/kg |

There was macroscopic evidence of haemolysis in the samples of the high-dose test animal, and no evidence of haemolysis in the baboon receiving the lower dose. From the long elimination half-life of the drug (12 - 20 hours) a single daily intravenous dose could suffice to sustain plasma levels of 50 µg/mℓ, the concentration shown to have persistent anti-HIV-1 properties in vitro. In spite of the very high drug concentrations in the plasma there did not appear to be any deleterious haemodynamic effects from intravenously administered BSSG-$(SO_4^-)_4$.

Stage 2 Test-Animal Code: B8/92

The Chronic Intravenous Administration of BSSG-$(SO_4^-)_4$ to a Chacma Baboon

The aim of this study was to evaluate the effect of maintaining a BSSG-$(SO_4^-)_4$ plasma level in excess of 50 µg/mℓ for about 1 month. In order to achieve this, $^{35}$S-isotope-labelled- BSSG-$(SO_4^-)_4$ was administered as a single daily intravenous injection into a 20 kg male Chacma baboon. The test animal received food and water without restriction. The animal was sedated by the intramuscular adminstration of ketamine (5 mg/kg) at 07h30 on Mondays to Fridays and given an intravenous (IV) injection of 100 mg (5mg/kg) of BSSG-$(SO_4^-)_4$ in saline solution.

On Tuesdays and Fridays blood samples were taken for blood chemistry, haematology and the measurement of trough plasma levels of the drug. These samples were taken prior to the administration of the drug, but at intervals as indicated in Figure 2 (which is a histogram of blood concentration levels against time) and Table 5. Blood samples were also taken 5, 10 and 15 minutes after drug adminstration in order to assess the peak plasma concentrations achieved. After 30 days the baboon was sacrificed and tissue samples were collected for testing.

RESULTS

The behavioral characteristics of the baboon during the period of treatment remained unchanged. Sleeping

habit, alertness and aggressive response to human proximity endured unchanged. Food and water intake remained normal.

The 24-hour post-dose-trough plasma levels of the drug are presented in Table 5 and Figure 2. In the interests of minimising blood taken from the animal the first 2 samples were omitted. The trough plasma levels in subsequent samples are seen to be in excess of 50 μg/mℓ, whereas the peak plasma levels achieved 5 minutes after administration of the drug reached up to 172 μg/mℓ. It is clear therefore that the daily average plasma level exposure to the drug is well in excess of 50 μg/mℓ (Figure 2 and Table 5). The trough plasma levels show a plateau effect over the study period, suggestive of a steady state. In terms of drug elimination from the plasma, the test animal thus remained uncompromised.

Table 5

| BSSG-$(SO_4^-)_4$ Plasma Levels and Free Haemoglobin Levels in the Plasma of a Chacma Baboon (B8/92) after the Intravenous Administration of BSSG-$(^{35}SO_4^-)_4$. | | |
|---|---|---|
| SAMPLING DATE | BSSG-$^{35}(SO_4^-)_4$ PLASMA LEVEL (μg/mℓ) | FREE HAEMOGLOBIN CONCENTRATION (mg/dℓ) |
| 28-2-92 | | 0.004 |
| 16-3-92 (Mon: Pre IV) | 0 | 0.005 |
| 24-3-92 (Tues: Pre IV) | 33.8 | 0.004 |
| 27-3-92 (Fri: Pre IV) | 60.3 | 0.004 |
| 31-3-92 (Tues: Pre IV) | 59.0 | 0.010 |
| 3-4-92 (Fri: Pre IV) | 56.0 | 0.003 |
| 3-4-92 (Fri: 5 min Post IV) | 172.0 | |
| 7-4-92 (Tues: Pre IV) | 71.0 | 0.005 |
| 10-4-92 (Fri: Pre IV) | 52.5 | |
| 10-4-92 (Fri: 10 min Post IV) | 149.5 | |
| 14-4-92 (Tues: Pre IV) | 65.0 | |
| 14-4-92 (Tues: 15 min Post IV) | 149.7 | |
| 15-4-92 (Wed: 4 hr Post IV) | 72.5 | |

Pre IV samples represent trough levels of BSSG-$(SO_4^-)_4$ whereas post IV samples represent peak levels. Post IV samples were taken after the IV administration of BSSG-$(SO_4^-)_4$ at the intervals indicated.

The Pharmacokinetics of Intravenous BSSG-$(SO_4^-)_4$ in the Chacma Baboon (B8/92)

The plasma elimination half-life of BSSG-$(SO_4^-)_4$ after intravenous administration to the baboon varied between 12 and 22 hours depending on the dosage (refer Table 4). A single daily intravenous injection of 5 mg of BSSG-$(SO_4^-)_4$/kg body mass over a month long period was sufficient to sustain plasma levels of the drug of 50-170 μg/mℓ, which concentrations are anti-viral in vitro. Measurement of $^{35}S$ Isotope in urine samples and faeces indicate that the major routes of excretion of BSSG-$(SO_4^-)_4$ are via the kidney and the liver. High levels of BSSG-$(SO_4^-)_4$ in the bile suggest that hepatic elimination may predominate. The in vitro anti-HIV-1 concentration of BSSG-$(SO_4^-)_4$ is sustained in vivo by a single daily intravenous injection of 5 mg/kg body mass.

Laboratory Test Results of Chacma Baboon B8/92

An autopsy was carried out which revealed no organ discoloration or change in organ texture. Tissue samples were taken for histological study and results of a pathology investigation are set forth hereunder. The investigation was based on the samples of a single test animal. The test animal referred to as B8/92 received

the compound according to the invention as described herein.

The histological findings regarding this animal were not severe and were fairly non-specific, and changes in histology could not necessarily be considered to be drug-related.

## Histological Investigation

Liver - There was a diffuse hydropic change although this was more obvious around the central veins and in the mid-zonal areas. This change may have been due to nutrition, starvation, metabolic disease or ingestion of toxins.

Kidneys - There were a few cortical tubules which were distended, and multifocal interstitial infiltration of lymphocytes. Some of the deep cortical glomeruli showed hyalinisation of Bowman's capsule, periglomerular fibrosis and lymphocyte infiltration. Occasional hyalin and granular casts were present in the medulla, and there was mild fibrosis of the interstitium. Distention of some of the tubules and some intersitial fibrosis found were probably due to a mild previous ascending urinary infection. These changes were mild.

Brain - There were no specific changes present in the brain.

Spleen - There were no congestion, active lymphoid tissue and increased neutrophils in the medulla. There were no neutrophils in the spleen but there were increased numbers of vacuolated macrophages, some collected in groups and scattered throughout the tissue.

Stomach - The mucosa were not affected. There was a neutrophilic vasculitis of most of the subserosal vessels, as well as neutrophilic leukostasis. Occasional vessels showed vasculitis. There were a few scattered cellular nodules containing corpora amylacea attached to the serosal surface, which may have been an artefact.

Lungs - There was prominent peripheral emphysema. There were multifocal areas of anthracosis and mild peribronchiolar lymphocyte cuffing, as well as mild neutrophilic leukostasis.

Heart - No specific changes were noted in the heart tissue.

Testes - The mucosa were not affected. The lamina propria in the tips of the villi were infiltrated by many macrophages containing cellular debris.

Bile Duct - No specific lesions were found. Similar structures to those described in the serosa of the stomach were present in the visceral peritoneum of the bile duct.

Urinary Bladder Wall - The urothelium was not affected. There were scattered foci of lymphocytes in the submucosa which were consistent with the findings of an ascending urinary tract infection as noted with regard to the kidneys. The bladder was not affected.

Pancreas - No specific changes were noted. The pancreatic lymph nodes showed similar foci of vacuolated macrophages, as seen in the spleen. (Stimulated macrophages, or accumulation of a storage product as seen in lysosomal storage diseases).

Portal vein - While it was difficult to identify the portal vein in the specimens, there were a number of smallish arteries and veins present, and these did not appear to be affected.

Gall bladder - There were mild neutrophilic leukostasis and occasional foci of lymphocyte infiltration in the submucosa. The gall bladder was not affected.

Colon - No specific changes were found in the colon.

## Bone Marrow Biopsy

A trephine biopsy-unilateral of suitable length and quality was carried out. Cellularity was approximately 40%, which value is slightly lower than that of an adult man. All haemopoeitic elements (megacaryocytes, erythroid precursors and granulocyte precursors) were morphologically within the normal range and were present in normal ratios.

Various pigment-filled macrophages were present. Special colouring techniques confirmed that the pigment comprised iron-haemocidarin. The lymphocytes and trabecula bone were also normal.

With the exception of mildly decreased cellularity all haemopoeitic elements appeared normal. The reason for the iron-filled macrophages was not clear.

Table 6

Baboon B8/92, Chemical Pathology Results of Baboon Serum Values after the Chronic Intravenous Administration of BSSG-$(SO_4^-)_4$ (5 mg/kg Body Mass).

| SERUM TEST AND UNITS | NORMAL RANGE IN CHACMA BABOONS | MON 16th | FRI 20th | TUE 24th | FRI 27th | TUE 31st | FRI 3rd | TUE 7th | FRI 10th | TUE 14th |
|---|---|---|---|---|---|---|---|---|---|---|
| Sodium (mmol/ℓ) | 142-150 | 144 | 145 | 145 | 148 | 146 | 145 | 146 | 142 | 151 |
| Potassium (mmol/ℓ) | 2.5-3.0 | 3.5 | 3.8 | 3.3 | 4.2 | 3.4 | 3.2 | 3.3 | 3.8 | 4.1 |
| Chloride (mmol/ℓ) | 103-111 | 104 | 110 | 104 | 103 | 101 | 102 | 98 | 96 | 106 |
| Total Carbon Dioxide (mmol/ℓ) | 23-31 | 25 | 28 | 15 | 18 | 25 | 16 | 23 | 28 | 21 |
| Anion gap (AG) (mmol/ℓ) | 7-17 | 15 | 7 | 26 | 27 | 20 | 27 | 25 | 18 | 24 |
| Urea (mmol/ℓ) | 5.3-8.7 | 6.2 | 7.8 | 5.0 | 7.9 | 7.5 | 6.9 | 8.8 | 6.2 | 7.9 |
| Creatinine (µmol/ℓ) | 68-98 | 96 | 98 | 106 | 124 | 88 | 134 | 140 | 116 | 121 |
| Total Protein (g/ℓ) | 63-74 | 70 | 73 | 70 | 70 | 71 | 71 | 76 | 65 | 67 |
| Albumin (g/ℓ) | 31-41 | 39 | 40 | 41 | 40 | 41 | 41 | 41 | 38 | 38 |
| Globulin (g/ℓ) | 28-37 | 31 | 33 | 29 | 30 | 30 | 30 | 35 | 27 | 29 |
| Cholesterol (mmol/ℓ) | 2.4-3.5 | 4.0 | 4.3 | 4.2 | 5.2 | 5.5 | 3.8 | 4.4 | 2.8 | 2.8 |
| Total Bilirubin (µmol/ℓ) | 0-6 | 3 | 3 | 3 | 7 | 3 | 4 | 4 | 22 | 11 |
| Conjugated Bilirubin (µmol/ℓ) | | 1 | 1 | 0 | 0 | 0 | 1 | 1 | 1 | 1 |
| Unconjugated Bilirubin (µmol/ℓ) | | 2 | 2 | 3 | 7 | 3 | 3 | 3 | 21 | 10 |
| Aspartate Transaminase (AST) (U/ℓ) | 25-64 | 26 | 30 | 54 | 88 | 47 | 54 | 50 | 101 | 74 |
| Alanine Transaminase (ALT) (U/ℓ) | 15-46 | 22 | 28 | 18 | 20 | 26 | 37 | 41 | 77 | 73 |
| Lactate Dehydrogenase (LD) (U/ℓ) | 185-478 | 299 | 254 | 390 | 831 | 286 | 560 | 483 | 815 | 913 |
| α-Glutamyl Transferase (GGT)(U/ℓ) | 14-40 | 38 | 36 | 32 | 30 | 30 | 36 | 37 | 35 | 37 |
| Alkaline Phosphatase (ALP) (U/ℓ) | 0-541 | 664 | 529 | 698 | 773 | 729 | 861 | 952 | 912 | 969 |

(The dates in the heading of Table 6 were in March/April 1992).

Samples were taken twice a week to a total of 9 samples. The sample of Monday, 16th March 1992, represents the baseline sample taken before initiation of BSSG-$(SO_4^-)_4$ administration. Samples correspond to the samples of Table 5 and Figure 2.

## Table 7

Haematology Results of Baboon Blood Samples after the Chronic Intravenous Administration of BSSG-$(SO_4^-)_4$ (5 mg/kg Body Weight) (Baboon B8/92).

| FULL BLOOD COUNTS | NORMAL VALUES IN MAN | MON 16th | FRI 20th | TUE 24th | FRI 27th | TUE 31st | FRI 3rd | TUE 7th | FRI 10th | TUE 15th |
|---|---|---|---|---|---|---|---|---|---|---|
| White Blood Cells ($x10^9/\ell$) | 4.1 -10.9 | 4.5 | 8.0 | 8.3 | 3.2 | 4.9 | 7.1 | 4.5 | 6.3 | 6.0 |
| Red Blood Cells ($x10^{12}/\ell$) | 4.53 - 6.48 | 5.91 | 5.49 | 5.71 | 5.61 | 5.94 | 5.61 | 5.76 | 5.25 | 5.97 |
| Haemoglobin (g/dℓ) | 13.05-17.95 | 14.8 | 13.9 | 14.6 | 13.7 | 14.1 | 13.2 | 13.6 | 12.5 | 11.8 |
| Haematocrit (%) | 40.14-53.86 | 43.4 | 41.2 | 42.0 | 40.5 | 43.1 | 41.5 | 41.9 | 38.1 | 36.9 |
| Mean Corpuscular Volume (fℓ) (MCV) | 80.14-93.86 | 73.4 | 75.0 | 73.8 | 72.2 | 72.5 | 74.0 | 72.6 | 72.6 | 74.1 |
| Mean Corpuscular Haemoglobin (pg)(MCH) | 27.05-31.95 | 25.0 | 25.0 | 25.69 | 24.5 | 23.8 | 23.5 | 23.6 | 23.9 | 23.7 |
| Mean Corpuscular Haemaglobin Concentration (%) (MCHC) | 29.56-35.44 | 34.0 | 34.0 | 35.0 | 33.9 | 32.7 | 31.8 | 32.5 | 32.8 | 32.0 |
| Red Cell Distribution Width ratio: cm/cm | 6.28-33.72 | 13.8 | 13.7 | | 13.0 | 12.7 | 13.0 | 12.3 | 12.3 | 12.2 |
| Platelets ($x10^9/\ell$) | 152.5-397.5 | 341 | 174 | 428 | 374 | 392 | 397 | 365 | 348 | 348 |
| Plateletcrit (%) | 0.17-0.33 | 0.24 | 0.14 | | 0.27 | 0.28 | 0.29 | 0.27 | 0.26 | 0.26 |
| Mean Platelet Volume ($\mu m^3$) | 6.1-8.9 | 7.0 | 7.8 | | 7.3 | 7.2 | 7.3 | 7.4 | 7.4 | 7.4 |
| Relative Values: | | | | | | | | | | |
| Neutrophils (%) | | 20.0 | 24.0 | 40.0 | 32.5 | 24.3 | 30.3 | 30.8 | 31.3 | 31.6 |
| Lymphocytes (%) | | 70.0 | 65.0 | 51.0 | 56.8 | 67.5 | 62.1 | 64.5 | 61.0 | 62.2 |
| Monocytes (%) | | 5.0 | 6.6 | 6.0 | 6.3 | 4.0 | 5.1 | 0.9 | 1.5 | 1.20 |
| Eosinophils (%) | | 4.0 | 4.3 | 3.0 | 4.0 | 3.8 | 2.4 | 3.5 | 5.80 | 4.90 |
| Basophils (%) | | | 0.2 | | 0.4 | 0.5 | 0.1 | 0.3 | 0.4 | 0.10 |
| Absolute Values: | | | | | | | | | | |
| Neutrophils ($x10^9/\ell$) | 2.06-7.45 | 0.91 | 1.93 | 3.31 | 1.04 | 1.19 | 2.15 | 1.39 | 1.97 | 1.90 |
| Lymphocytes ($x10^9/\ell$) | 1.52-3.97 | 3.16 | 5.18 | 4.22 | 1.82 | 3.31 | 4.41 | 2.90 | 3.84 | 3.73 |
| Monocytes ($x10^9/\ell$) | 0.21-0.79 | 0.23 | 0.53 | 0.50 | 0.20 | 0.2 | 0.36 | 0.04 | 0.09 | 0.07 |
| Eosinophils ($x10^9/\ell$) | 0.05-0.45 | 0.19 | 0.34 | 0.25 | 0.13 | 0.19 | 0.17 | 0.16 | 0.37 | 0.29 |
| Basophils ($x10^9/\ell$) | 0.04-0.16 | | 0.02 | | 0.01 | 0.02 | 0.01 | 0.01 | 0.03 | 0.01 |

The sample of Monday, 16th March 1992, represents the baseline sample taken before initiation of BSSG-$(SO_4^-)_4$ administration.
The samples correspond to samples of Table 5, Table 6 and Figure 2.

*In vitro* study: Evaluation of the Anticoagulant Effects of BSSG-$(SO_4^-)_4$

The measurement of the prothrombin time in vitro showed that BSSG-$(SO_4^-)_4$ did not affect blood clotting at a concentration of 50 μg/mℓ.

The results are set out in Table 8.

Table 8

| The In Vitro Evaluation of Prothrombin Time at 50 μg BSSG-$(SO^-_4)_4$/mℓ Plasma. | |
|---|---|
| Substance | Prothrombin Time (seconds) |
| Saline control | 15.3-15.6 |
| BSSG-$(SO_4^-)_4$ in plasma (50 μg/mℓ) | 14.8-14.3 |
| Heparin (8 International Units (IU)/mℓ) | 180 |

Prothrombin Time was measured according to standard techniques using $CaCl_2$, citrated-plasma and prothrombin. The prothrombin time of BSSG-$(SO_4^-)_4$ at 50μg/mℓ is similar to that of the saline control.

Chemical Pathology Investigation - Baboon B8/92 (refer Table 6)

The electrolytes were seemingly unaffected, except for the bicarbonate, which at times indicated a slight acidosis. The acidosis was substantiated by the increase of the anion gap (AG). The increase in the AG can however also be attributed to the fact that the substance administered was also an anion, which would increase the AG. This may be the reason why the AG increased after treatment was started.

There is no clear reason for the high creatinine level as there was no consistent rise-in the values, as would have been expected were the substance to be nephrotoxic. The fluctuations in the values can better be ascribed to changes in the level of hydration of the animal as well as interferences of substances other than creatinine with the Jaff colour reaction, used for the determination of creatinine.

The initial high cholesterol levels, which were present from the beginning, but returned to normal values, were difficult to explain. The high values cannot be due to cholestasis as there are no other parameters (such as high α-glutamyl transferase (GGT)) indicating cholestasis.

The liver enzymes were difficult to interpret as there was no consistent pattern. The increase in the lactate dehydrogenase (LD) and aspartate transaminase (AST) level could possibly have been due to haemolyses, especially as indicated in the last two specimens. The rise in unconjugated bilirubin supported the possibility of haemolysis. The rise in alanine transaminase (ALT) may indicate slight liver cell damage. The slight rise in the LD and AST levels on Friday 27th March 1992, may also have reflected a haemolytic incident.

As the GGT levels were normal, the high alkaline phosphatase (ALP) levels must have had an origin other than the liver, which origin may have either been bone or the gastro-intestinal tract. The reason for the increase in ALP could however not be deduced from the results available.

In spite of all the speculation there was no direct proof that the changes occurring in time were due to direct toxicity of the substance.

Haematology Investigation - Baboon B8/92 - (refer Table 7)

Haemoglobin (Hb) and haematocrit (Hct) values showed a general decrease from the base-line Hb and Hct values (14.8 to 12.5g/dℓ and 43.4 to 36.9%), respectively) which correlated with the biochemical evidence of mild haemolysis during drug administration. (No reticulocyte counts or haptoglobin values were available for additional confirmation of haemolysis).

Although no compensatory increase in erythropoiesis in the bone marrow was found, bone marrow iron stores were increased, a further finding that supported haemolysis.

Mean corpuscular volume (MCV) and mean corpuscular haemoglobin (MCH) were below the normal levels compared to human adult values and fell within the hypochromic microcytic range as found in e.g. anaemia of

chronic disorders in the adult human being.

Total white blood cell count (WBC) generally showed an initial increase after adminstration of the compound, but remained within the normal range, compared to adult human WBC values.

There was also an inverse relationship between lymphocyte and neutrophil counts (L:N ratio) at basal levels before administration, compared to normal adult human ratios.

The L:N ratio tended to decrease after administration, rising again (5th determination) and thereafter remaining below the basal ratio level. This was mainly due to the relative increase in neutrophil counts following administration.

Absolute neutropaenia was present at basal level before administration. A slight transient increase followed after administration which fell to the low initial value at the 4th determination, thereafter remaining slightly raised. However, no absolute neutophilia was found compared to normal adult human neutrophil count ranges.

The lymphocyte count showed a transient absolute increase after commencement of treatment, but relatively less than that of the neutrophil count. The lymphocyte count decreased to a low normal value (compared to adult human values) along with a decrease in neutrophil count at the 4th determination, and thereafter returned to near basal level.

The monocyte count remained within normal limits until the 6th determination, whereafter an absolute monocytopaenia was recorded which remained below normal adult human levels up to the end.

No abnormality in either eosinophil or basophil counts was observed.

Platelet count was within normal range throughout the whole series of determinations, compared to adult human values.

A decrease in platelet count (not below normal levels) occurred once only after initial administration of the drug (2nd determination), followed by a compensatory slight increase in platelet count (within normal limits). Thereafter the platelet count remained normal. This could have been due to either a temporary increase in platelet destruction in the spleen or to a temporary inhibition of platelet production in the bone marrow immediately following drug administration. However, it was unlikely that platelets were peripherally destroyed owing to drug administration, because no compensatory increase of megakaryopoiesis was documented on bone marrow biopsy.

The significance of this transient fall in platelet count could not be evaluated from only one observation.

## Summary

Haemoglobin (Hb) and haematocrit (Hct) values had an overall decreasing trend that suggested slight haemolysis, supported by biochemical and bone marrow iron findings.

There were slightly hypochromic microcytic red cell indices compared to adult human values. (With the increased iron stores, this finding was consistent with anaemia of chronic disease in adult humans).

There was a slight increase in total white cell count but within normal range following drug administration, owing to increase in both neutrophil (N) and lymphocyte (L) counts.

The basal L:N ratio was inverted as compared to adult human L:N ratios.

There was basal absolute neutropaenia with relative neutrophilia following drug administration.

There was transient absolute lymphocytosis following initial drug administration.

Absolute monocytopaenia followed after the second week of drug administration and was still present at the end.

There was a transient decrease in platelet count (but still within normal range) following initial drug administration.

## *In Vitro* Studies: Evaluation of the Cytotoxicity of β-Sitosteryl β-D-Glucoside *Tetra*-sulphates

β-Sitosterol (BSS), β-sitosteryl β-D-glucoside (BSSG), and B-sitosteryl β-D-glucoside tri- and tetra-sulphates (BSSG-$(SO_4^-)_4$) were evaluated in tissue culture for cytotoxicity.

## Cells and Culture

Peripheral blood lymphocytes were prepared from normal blood. Cells were cultured for 3 days in complete medium [(RPMI, a commercially available so-called standard salt solution) + 10 % foetal calf serum (FCS) + antibodies] in the presence/absence of phytohemagglutinin (PHA). Duplicate wells were set up. Incubation was at 37°C and 5 % $CO_2$.

On the third day, cell viabilities were determined using the Trypan Blue exclusion test.

Solubilization of Products

Those products which were water soluble, were made up at 8 mg/mℓ stocks in sterile distilled water and diluted 1:10 in RPMI medium and again diluted 1:10 directly in the wells containing the cells in complete medium. The final concentrations were thus 80 μg/mℓ. For the lower dilutions, the 8 mg/mℓ stock was diluted 1:2 or 1:4 or 1:8 in water before being diluted in the RPMI medium.

For those products which were DMSO-soluble, the stocks were made'up at 8 mg/mℓ in 100 % DMSO. This stock was diluted 1:10 in 100 % FCS and diluted 1:10 directly in the wells containing the cells in RPMI medium only. The final concentrations were thus 10 % FCS and 1 % DMSO. For the lower dilutions, the 8 mg/mℓ stock was diluted 1:2 or 1:4 or 1:8 with DMSO before the FCS dilution step.

For the BSSG- $(SO_4^-)_4Na^+_4$ a stock of 20 mg/mℓ was made in 100 % ethanol (EtOH). This stock was diluted in water to yield a working stock of 2 mg/mℓ. The 2 mg/ml stock was diluted 1:20 or 1:10 or 1:5 or 1:2.5 in water and again 1:10 in the wells to give the 10, 20, 40 or 80 μg/mℓ final concentrations respectively.

The experimental protocol is as set out below.

a) Cells + medium.
b) Cells + PHA.
c) Cells + PHA + 1 % $H_2O$.
d) Cells + PHA + 1 % DMSO.
e) Cells + PHA + 0.1 % EtOH.
f) Cells + PHA + 0.2 % EtOH.
g) Cells + PHA + 0.4 % EtOH.
h) Cells + PHA + BSS (10, 5, 2.5 and 1.25 μg/mℓ).
i) Cells + PHA + BSSG (10, 5, 2.5 and 1.25 μg/mℓ).
j) Cells + PHA + BSSG-$(SO_4^-)_4$ (80, 40, 20 and 10 μg/mℓ).

The results are set out in Table 9.

Table 9

| Evaluation of the Cytotoxicity of BSS, BSSG and BSSG-$(SO_4^-)_4$ on Peripheral Blood Lymphocytes In Vitro. | |
|---|---|
| | % Viability |
| Cells + medium | 85 |
| Cells + PHA | 97 |
| Cells + PHA + 1 % $H_2O$ | 95 |
| Cells + PHA + 1 % DMSO | 86 |
| Cells + PHA + 0.1 % EtOH | 93 |
| Cells + PHA + 0.2 EtOH | 89 |
| Cells + PHA + 0.4 EtOH | 88 |
| Cells + PHA + BSS 10 μg/mℓ | 82 |
| 5 μg/mℓ | 92 |
| 2.5 μg/mℓ | 81 |
| 1.25 μg/mℓ | 89 |
| Cells + PHA + BSSG 10 μg/mℓ | 90 |
| 5 μg/mℓ | 87 |
| 2.5 μg/mℓ | 89 |
| 1.25 μg/mℓ | 91 |
| Cells + PHA + BSSG-$(SO_4^-)_4$ 80 μg/mℓ | 83 |
| 40 μg/mℓ | 82 |
| 20 μg/mℓ | 85 |
| 10 μg/mℓ | 80 |

% Viabilities = means of 4 counts.

As can be seen from Table 9, the viability of test cells exhibited no tendency to toxicity with progressive increase in BSSG-$(SO_4^-)_4$ concentration in the test media, from 10 to 80 μg/mℓ.

*In Vivo* Anti-FIV Tests

Materials and Methods

A cat which had been experimentally infected with a strain of feline immunodeficiency virus (FIV) using the blood of a naturally infected cat was used for this experiment. The cat was monitored for seroconversion using a commercial kit for the measurement of FIV-specific bodies and, at an appropriate time, it was treated with BSSG-$(SO_4^-)_4$ by weekly sub-cutaneous injections at a dose of 5 mg/kg body mass. The haematological and immunological parameters were monitored during the dosage period as described below.

Blood Analysis

Clotted Blood

A specimen of blood was used to determine the antibody titer to FIV using a commercial kit (CITE Combo,

Indexx Corporation, Portland). The serum was diluted with saline solution and each serum diulation was tested in order to determine the highest dilution yielding positive results. This dilution was subsequently used as the cut-off titer of positivity. The titer of antibodies was determined at regular intervals during the treatment period.

EDTA Whole Blood

Whole blood was diluted by 50% with RPMI medium and layered on to Histopaque (Sigma) and centrifuged at 200 gravities for 25 minutes. The cells at the interface were collected and washed three times with RPMI. These cells represented the mononuclear cells of the blood and were used for determining the percentage of T-cells and the subsets thereof as described hereunder.

Cells (1 x $10^6$) were incubated in test tubes with aliquots of monoclonal antibody reactive with CD3, CD4 and CD8 antigens. The mixtures of different antibodies were conducted as follows:
CD3-FITC + CD4-PE
CD3-FITC + CD8-PE

The tubes were incubated for 20 minutes at room temperature and following two washes with phosphate-buffered saline solution (PBS), the cells were fixed with 400 $\mu\ell$ PBS containing 0.5% formaldehyde.

Cell fluoresence was assessed using a FACScan cytometer. FL1 (fluoroscein, FITC) was measurred using a 530/30 nm band pass filter and the FL2 (phycoerythrin, PE) was measured with a 585/42 nm band pass filter. The analysis was conducted using the LYSIS II software programme after having set a lymphocyte gate on the FSC versus SSC scattergram. All the results were expressed as percentage positive lymphocytes, i.e. cells which co-expressed CD3 and CD4 or CD3 and CD8 markers. In this way, $CD4^+$ T cells, and in a similar fashion, $CD8^+$ NK cells, were excluded from the $CD3^+$ $CD8^+$ T-suppressor cells.

Absolute cell numbers were calculated from basic haematology conducted on an aliquot of the whole blood. From the percentage lymphocyte differential counts, absolute values for positive cells could be calculated. This was conducted for all subsets analyzed.

RESULTS

Serum Antibody Titer

As shown in Table 10 hereunder, the serum titer of antibodies detected with the commercial CITE Combo kit was constant at a 1:512 titer prior to the initiation of treatment. Within two weeks of the commencement of BSSG-$(SO_4^-)_4$ treatment, the serum titer declined to 1:256 and at the last follow-up (13 weeks post-treatment), the titer had dropped to 1:128, indicative that the antigen load (and hence viral load) had been decreased by the treatment, and the consequence thereof was the decline in antibody synthesis (Table 10).

TABLE 10: ANTIBODY TITER OF CAT TREATED WITH WEEKLY INJECTIONS OF BSSG-$(SO_4^-)_4$

| Time (weeks) | Antibody Titer |
|---|---|
| 0 | 1:512 |
| 6 | 1:512 |
| 12 | 1:256 |
| 17 | 1:256 |
| 23 | 1:128 |

The treatment was initiated at 10 weeks.

Lymphocyte Subsets:

As shown in Table 11 hereunder, the cat showed increasing leucocyte counts as well as increasing total lymphocyte counts. When the lymphocytes were analysed by monoclonal antibodies and flow cytometry, the $CD4^+$ cells exhibited increased cell counts once the treatment had been initiated indicative that the viral load had been removed and hence that the CD4 cells could recover from viral infectivity. This increase in $CD4^+$ cell

counts was accompanied by increases in the CD3+ cell counts as well as the CD8+ cell counts.

TABLE 11: HAEMATOLOGICAL AND IMMUNOLOGICAL PARAMETERS IN THE CAT TREATED WITH WEEKLY INJECTIONS OF BSSG-($SO_4^-$)$_4$ SUB-CUTANEOUSLY:

| Time (weeks) | WBC (x$10^9$/ℓ) | Lymph. (μℓ) | CD3+ (μℓ) | CD4+ (μℓ) | CD8+ (μℓ) |
|---|---|---|---|---|---|
| 0 | 11.8 | 2600 | 1742 | 460 | 1115 |
| 6 | 13.2 | 2380 | 1219 | 350 | 721 |
| 12 | 14.7 | 2940 | 964 | 315 | 432 |
| 17 | 14.0 | 3220 | 2035 | 763 | 1066 |
| 23 | 15.5 | 3990 | 2894 | 850 | 1610 |

The treatment was initiated at 10 weeks.
WBC: White blood cells
Lymph.: Lymphocytes

The relationship of antibody titer to absolute CD4+ cell counts is shown in Figure 3. From Figure 3 it can be seen that, once the treatment was initiated, the titer of antibodies specific to the FIV declined and, at the same time, the absolute cell counts of T-helper cells increased, indicative that the viral load was suppressed, thereby allowing the CD4+ cells to recover.

General Conclusions of *In Vitro* and *In Vivo* Studies

The pharmacokinetics of BSSG-($SO_4^-$)$_4$ in the Chacma baboon were such that anti-HIV-1 concentrations of the drug were sustainable after a single daily intravenous administration. Maintenance of anti-HIV-1 levels in the test animal for a month-long period resulted in laboratory data which suggested that the drug may have acceptable levels of toxicity in terms of clinical use. The in vitro efficacy and suppression of FIV viral load in the cat by BSSG-($SO_4^-$)$_4$ and its apparent in vivo compatibility augered well for its evaluation as an anti-viral therapeutic agent in man.

The Chemical Synthesis of Steryl and Spiroketyl Steroid Glycoside Sulphates

By way of example a method is described for the synthesis of BSSG-($SO_4^-$)$_4$$Na^+_4$ from BSSG. The method may be used in principle for the sulphation of the glycosides of other sterols (for example chlosteryl glucoside) and glycosides of spiroketal steroids (for example diosgenyl glucoside, tigogenyl glucoside and hecogenyl glucoside). The glycosides of the sterols and spiroketal steroids may be prepared by the coupling of the saccharide unit (for example glucose) to the 3-hydroxy group of the aglycone by means of a suitable procedure such as the Königs- Knorr synthesis (Königs W., Knorr E., Ber. 34, 957, 1901).

Synthesis of BSSG-($SO_4^-$)$_4$$Na^+_4$

To an ice-cold solution of chlorosulphonic acid (10 mℓ, 0.15M) in pyridine (100 mℓ) was added sistosteryl β-D-glucoside (4 g, 0.0069M). After dissolution the mixture was allowed to stand at 90-100°C for 1 hour before cooling, and then it was poured into ice-cold aqueous sodium hydroxide (200 mℓ, 2M) in a separating funnel and slowly inverted 40 times. On settling, two phases formed, the lower, aqueous, phase being drawn off. Methanol (175 mℓ) was slowly stirred into the aqueous phase, whereupon a precipitate formed, which was collected by filtration. The filter cake was dissolved in water (525 mℓ) whereupon methanol (350 mℓ) was slowly added with stirring and the mixture filtered as previously. The filter cake was again dissolved in water (200 mℓ) and methanol (150 mℓ) slowly added while stirring. The mixture was filtered as before and the filter cake washed with aqueous methanol (50 mℓ, 40%). The filter cake was dissolved in water (50 mℓ) and lyophylised to provide β-sitosteryl β-D-glucoside tetra-sulphate sodium salt (3.5 g, 0.0036M, 51% yield) as a white powder with an analysis for C, 37.54%; H, 4.8 6%; and S, 12.27%. $C_{35}H_{56}O_{18}S_4Na_4$ requires C, 42.68%; H, 5.73% and S, 13.02%.

Similar procedures were used to prepare CHOG-($SO_4^-$)$_4$$Na^+_4$ and DG-($SO_4^-$)$_4$$Na^+_4$.

The Properties and Identification of β-Sitosteryl β-D- Glucoside *Tetra*-Sulphate Sodium Salt [BSSG-$(SO_4^-)_4Na^+_4$]

The product was a white powder that dissolved in cold water. Hot aqueous solutions of concentration above 10 g/ℓ formed clear gels on cooling. The product was insoluble in organic solvents.

Chromatography

The product was characterised by thin layer chromatography (TLC) using Silica Gel G coated glass plates (Merck 5715).

The TLC plates were activated at 90°C for 60 minutes and spotted with 20 μg of BSSG-$(SO_4^-)_4$ as an aqueous solution and developed with three different solvent mixtures.

The TLC spots were visualised by spraying with anisaldehyde- sulphuric acid spray reagent (Stahl E., Kaltenbach U., J. Chromatog., 5, 351, 1961).

After the plates were sprayed and a current of hot air was applied, the product spot appeared as a purple spot.

The TLC plates were developed for 7 cm with the following solvents, the product appearing as a single spot in each case:

chloroform, ethanol, water (33:50:17 by volume)
Rf BSSG-$(SO_4^-)_4$ = 0.55
benzene, methyl propyl ketone, ethanol, water (2:3:4:2)
Rf BSSG-$(SO_4^-)_4$ = 0.50
chloroform, acetic acid, water (6:2:2)
Rf BSSG-$(SO_4^-)_4$ = 0.29

The Hydrolysis of BSSG-$(SO_4^-)_4$ Sodium Salt

BSSG-$(SO_4^-)_4$ can be hydrolysed completely to several components by aqueous hydrochloric acid (0.5 N) at 90°C within 10 minutes. Five hydrolysis products can be identified by the aforementioned TLC conditions using the developing solvent chloroform:ethanol:water (33:50:17) with Rf values of 0.69; 0.76; 0.84; 0.92; and 0.98 respectively. β-Sitosteryl β-D-glucoside has an Rf value of 0.98, this corresponding to the end product of mild acid hydrolysis of BSSG-$(SO_4^-)_4$. Spots of Rf 0.69; 0.76; 0.84; and 0.92 are considered to correspond to various combinations of mono-, di- and tri-sulphates of β-sitosteryl β-D-glucoside, several structural combinations of each being possible. All the products of hydrolysis of BSSG-$(SO_4^-)_4$ displayed the same purple colour on visualisation with the anisaldehyde-sulphuric acid spray reagent.

The Determination of Sulphate in BSSG-$(SO_4^-)_4$ Sodium Salt by Gravimetric Analysis

BSSG-$(SO_4^-)_4$ sodium salt was precipitated from an acidic aqueous solution by barium chloride. The acidic conditions used in the determination were shown to be too mild to cause hydrolysis of the sulphate groups of BSSG-$(SO_4^-)_4$. The BSSG-$(SO_4^-)_4$ was assumed to precipitate as the di-barium salt.

Procedure

BSSG-$(SO_4^-)_4$ sodium salt (100 mg) was dissolved in hot water (20 mℓ), cooled and had hydrochloric acid added thereto (1 mℓ, 1M). Immediately an aqueous solution of barium chloride was added (4 mℓ, 10% $BaCl_2.2H_2O$). The mixture was then centrifuged and the supernatant decanted to waste. The precipitate was washed with water (20 mℓ). The precipitate was vacuum dried to a constant mass.

Results

$$\frac{\text{Mass of precipitate}}{\text{Mass of BSSG} - (SO_4^-)_4Na^+_4} = \frac{113\text{ mg}}{100\text{ mg}}$$

$$\text{Mass Ratio} = 1.130$$

$$\text{Molecular weight ratio of expected product} = \frac{\text{BSSG} - (SO_4^-)_4\,Ba^{2+}_2}{\text{BSSG} - (SO_4^-)_4Na^+_4} = \frac{1166.5}{984}$$

$$\text{Molar ratio} = 1.186$$

The practical ratio is therefore 95.3% of the expected theoretical ratio, suggesting that BSSG-$(SO_4^-)_4$ is a tetra-sulphate.
BSSG-$(SO_4^-)_4$ sodium salt was hydrolysed under strong acidic hydrolysis and the sulphate anion ($SO_4^-$) determined as $BaSO_4$.

Procedure

BSSG-$(SO_4^-)_4$ sodium salt (100 mg) was dissolved in hot water (5 mℓ). To this concentrated hydrochloric acid (10M, 5 mℓ) was added. The solution was heated in a hot water bath at 100 °C for 15 minutes. The solution was left for 24 hours at room temperature and filtered. While the solution was mixed slowly, aqueous barium chloride was added (10%, 4 mℓ). The precipitate was isolated by centrifugation and washed twice with water (20 mℓ). The precipitate was vacuum dried to constant mass.

Results

The number of $SO_4^-$ groups calculated in 3 assays were 3.60, 3.75 and 3.35. The average number of sulphate groups, 3.57, indicated the presence of four sulphate groups/molecule of β-sitosterol β-D-glucoside.

Elemental Analysis

The molecular formula of β-sitosterol β-D-glucoside tetra-sulphate tetra-sodium salt corresponds to $C_{35}H_{56}O_{18}S_4Na_4$ with a molecular mass of 985.0166 atomic mass units. Computed elemental content gave the following values: C= 42.68%, H = 5.73%, O = 29.24%, S = 13.02%, Na = 9.34%.
Elemental analysis of this product gave the following data: C = 37.54%, H = 4.86%, S = 12.27%. These values are indicative of at least four sulphur atoms in the molecule. These sulphur atoms were incorporated by a standard method for the sulphation of saccharides, (refer the chemical synthesis of BSSG-$(SO_4^-)_4$ described above).

List of Abbreviations

Abbreviations used above in this specification are as follows:

- HIV-1 = Human Immunodeficiency Virus Type 1.
- $P_{24}$ = HIV core protein.
- FIPV = Feline Infectious Peritonitis Virus.
- BSS = β-Sitosterol.
- BSS-$SO_4^-$ = β-Sitosteryl sulphate.
- BSSG = β-Sitosteryl β-D-glucoside.
- BSSG-$(SO_4^-)_4$ = β-Sitosteryl β-D-glucoside tetra-sulphate.
- CHO = Cholesterol.
- CHO-$SO_4^-$ = Cholesteryl sulphate.
- CHOG = Cholesteryl β-D-glucoside.
- CHOG-$(SO_4^-)_4$ = Cholesteryl β-D-glucoside tetra-sulphate.
- D = Diosgenin.
- Lymph. = Lymphocytes.
- D-$SO_4^-$ = Diosgenyl sulphate.
- DG = Diosgenyl β-D-glucoside.
- DG-$(SO_4^-)_4$ = Diosgenyl β-D-glucoside tetra-sulphate.
- PBL = Peripheral Blood Lymphocytes.
- PHA = Phytohemagglutinin.
- IL-2 = Interleukin 2.
- DMSO = Dimethylsulphoxide.
- AZT - 3'-azido-3'-deoxythymidine.
- RNA = Ribonucleic Acid.
- ELISA = Enzyme Linked Immunosorbent Assay
- Rf = Relative Migration Factor
- IV = Intravenous
- AG = Anion Gap
- AST = Aspartate Transaminase

ALT = Alanine Transaminase
LD = Lactate Dehydrogenase
GGT = α-Glutamyl Transferase
ALP = Aklaline Phosphatase
WBC = White Blood Cell
RBC = Red Blood Cell
Hb = Haemoglobin
Hct = Haematocrit
MCV = Mean Corpuscular Volume
MCH = Mean Corpuscular Haemoglobin
MCHC = Mean Corpuscular Haemoglobin Concentration
N = Neutrophil
L = Lymphocyte
FCS = Foetal Calf Serum
EtOH = Ethanol
g = gram
ℓ = litre
mℓ = millilitre
DNA = deoxyribonucleic acid
TLC = thin layer chromatography
RPMI = Rosewell Park Memorial Institute
FITC = fluorescein insothiocyanate
PE = phycoerythrin
FIV = feline immunodeficiency virus
PBS = phosphate-buffered saline solution
FL1 = fluorescence 1
FL2 = fluorescence 2

Cholesteryl 3-β-D-glucopyranoside, sitosteryl 3-β-D-glucopyranoside (referred to elsewhere in this specification respectively as cholesteryl β-D-glucoside and sitosteryl β-D-glucoside) and their respective tetra-sulphates can be represented by the following structural formula, Formula I:

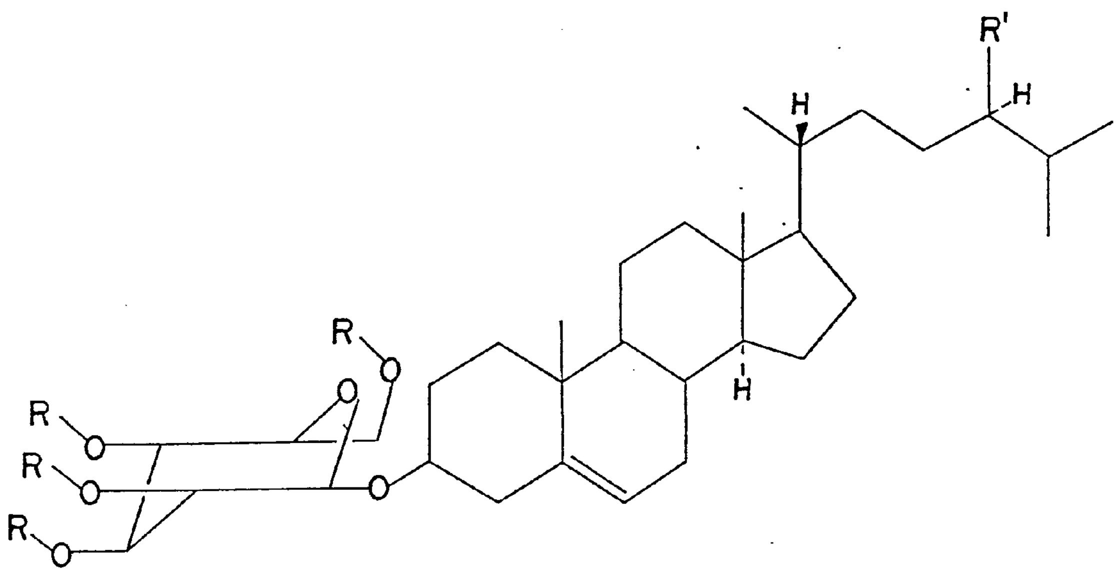

Formula 1

in which

R = R' = H for cholesteryl 3-β-D-glucopyranoside.

R = H and R' = $C_2H_5$ for sitosteryl 3-β-D-glycopyranoside

R = $-SO_3^-$ for the tetra-sulphates of both said compounds .

In turn, diosgenyl 3-β-D-glucopyranoside (referred to elsewhere in the specification as diosgenyl β-D-glucoside) and its tetra-sulphate can be represented by the following structural formula, Formula II:

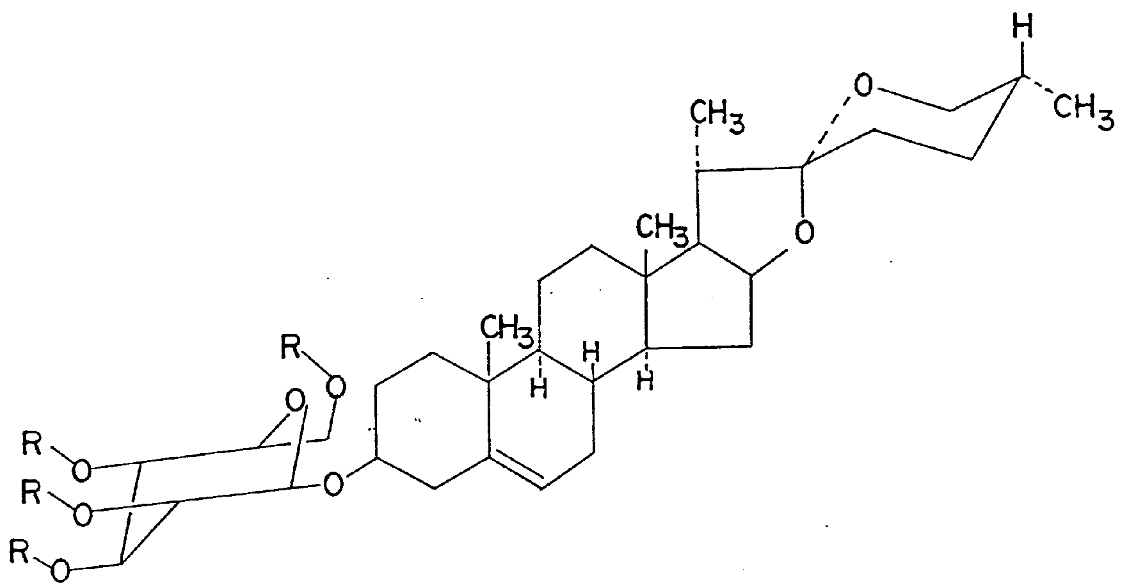

Formula II

in which:

R = H for diosgenyl β-D-glucopyranoside.

R = $-SO_3^-$ for the tetra-sulphate thereof.

Finally, hecogenyl 3-β-D-glycopyranoside (referred to elsewehere in the specification as hecogenyl β-D-glucoside) and its tetra-sulphate can be represented by the following structural formula, Formula III:

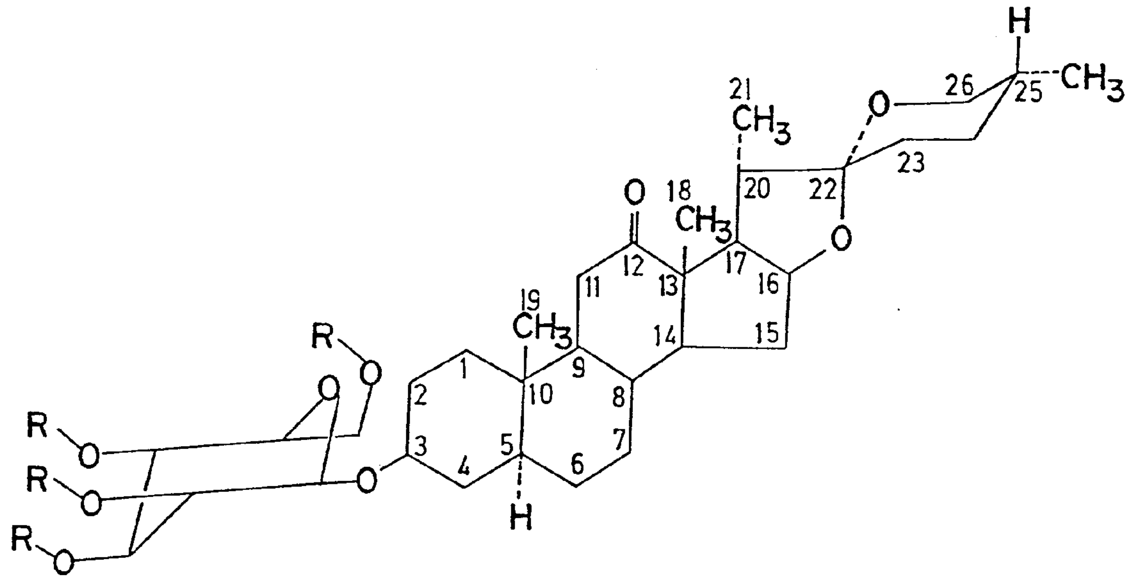

Formula III

in which:

R= H for hecogenyl 3-β-D-glucopyranoside.

R = $-SO_3^-$ for the tetra-sulphate thereof.

It should be noted that:

with regard to Formulae I and II the 5(6) double bond may be saturated to provide 5α-H analogues, these being 5α,6-dihydrocholesteryl (i.e. 5α-cholestanyl), 5α-sitostanyl and tigonyl respectively;

in Formula I the 7(8)-ene and/or 22(23)-ene analogues may be present;

R' in Formula I may be a methyl or an ethyl group or their epimers (i.e. Rβ);

in Formula I, R' together with the 24-H may be a methylene group (i.e. $=CH_2$) or an ethylidene cis or trans group; and

C-25 in Formulae II and III may be epimeric.

## Claims

1. A chemical compound characterised in that it comprises a sulphated glycoside selected from sulphated steryl glycosides and sulphated spiroketyl steroid glycosides, the sulphated glycoside having:
   a hydrophobic portion selected from sterol aglycone groups having steryl groups and spiroketal steroid aglycone groups having spiroketyl steroid groups, each said aglycone group having a 3-oxy-glycoside linkage;
   at least one saccharide unit coupled to said aglycone group via the 3-oxy-glycoside linkage of said aglycone group; and
   at least one sulphate group which is a sulphated ester of a hydroxyl group which forms part of said saccharide unit.

2. A compound as claimed in claim 1, characterised in that it is a sulphated steryl glycoside whose aglycone group has an aglycone moiety which is a phytosterol selected from cholesterol, β-sitosterol, campesterol, stigmasterol and spinasterol.

3. A compound as claimed in claim 1, characterised in that it is a sulphated spiroketyl steroid glycoside whose aglycone group has an aglycone moiety selected from diosgenin, tigogenin and hecogenin.

4. A compound as claimed in any one of the preceding claims, characterised in that each saccharide unit is selected from monosaccharide units derived respectively from pentoses and hexoses, and monosaccharide units which are glucoside units.

5. A compound as claimed in claim 4, characterised in that each saccharide unit is a glucoside unit which is sulphated and is selected from the mono-, di-, tri- and tetra-sulphates of glucoside units.

6. A compound as claimed in any one of the preceding claims, characterised in that it is in acid form.

7. A compound as claimed in any one of claims 1 - 5, characterised in that it is in the form of a salt having at least one sulphate anion, each sulphate anion being associated with a counterion selected from cations consisting of sodium-, ammonium-, substituted ammonium-, potassium-, magnesium- and calcium cations.

8. A compound according to claim 1 which is:
β-sitosteryl β-D-glucoside tetra-sulphate;
cholesteryl β-D-glucoside tetra-sulphate;
campesteryl β-D-glucoside tetra-sulphate;
stigmasteryl β-D-glucoside tetra-sulphate;
spinasteryl β-D-glucoside tetra-sulphate;
diosgenyl β-D-glucoside tetra-sulphate;
tigogenyl β-D-glucoside tetra-sulphate; or
hecogenyl β-D-glucoside tetra-sulphate.

9. A compound according to claim 1, which is a sodium salt of:
β-sitosteryl β-D-glucoside tetra-sulphate;
cholesteryl β-D-glucoside tetra-sulphate; or
diosgenyl β-D-glucoside tetra-sulphate;

10. A process for the preparation of a compound as claimed in any one of the preceding claims, which comprises sulphating a steryl glycoside or a spiroketyl steroid glycoside.

11. A pharmaceutical composition which comprises a compound as claimed in any one of claims 1 - 9, in association with a pharmaceutically acceptable carrier or coating.

12. A compound according to any one of claims 1 to 9 for use as a medicine or a medicament.

13. Use of a compound according to any one of claims 1 to 9 in the preparation of a composition, medicine or medicament for treating or preventing a viral infection in a human or animal subject.

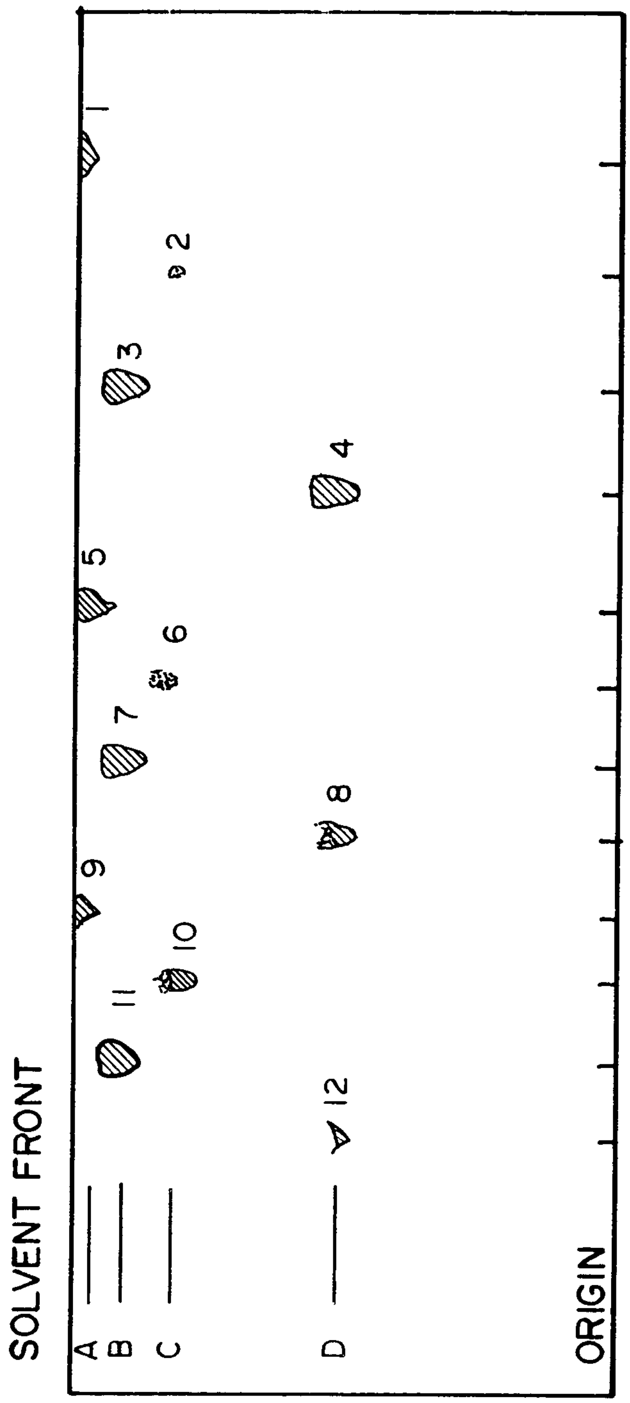


FIG 1

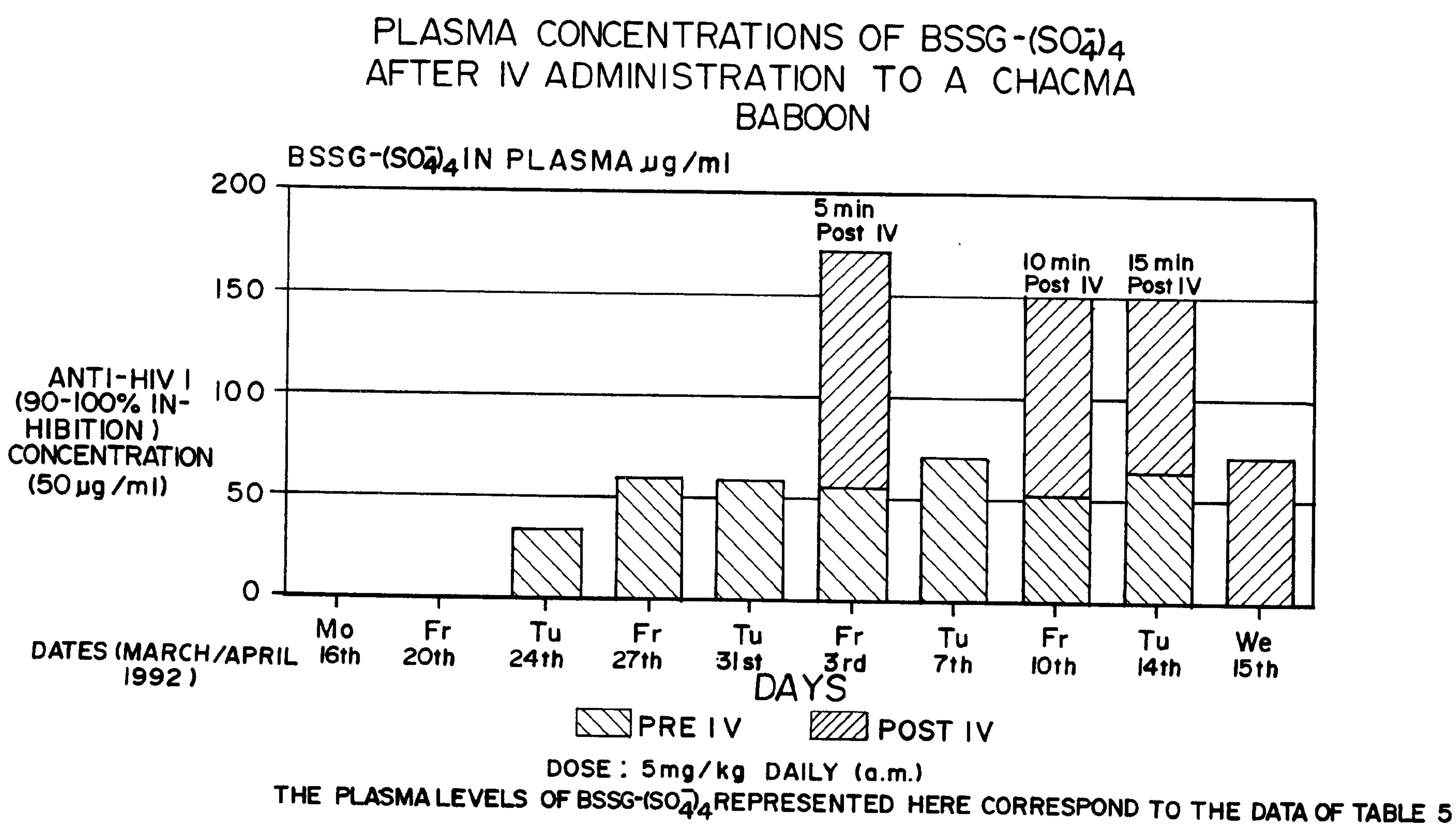
PLASMA CONCENTRATIONS OF BSSG-$(SO_4^-)_4$
AFTER IV ADMINISTRATION TO A CHACMA
BABOON
BSSG-$(SO_4^-)_4$ IN PLASMA µg/ml
200
150
100
50
0
ANTI-HIV I
(90-100% IN-
HIBITION)
CONCENTRATION
(50 µg/ml)
5 min
Post IV
10 min
Post IV
15 min
Post IV
DATES (MARCH/APRIL
1992)
Mo
16th
Fr
20th
Tu
24th
Fr
27th
Tu
31st
Fr
3rd
Tu
7th
Fr
10th
Tu
14th
We
15th
DAYS
PRE IV
POST IV
DOSE : 5mg/kg DAILY (a.m.)
THE PLASMA LEVELS OF BSSG-$(SO_4^-)_4$ REPRESENTED HERE CORRESPOND TO THE DATA OF TABLE 5

FIG 2

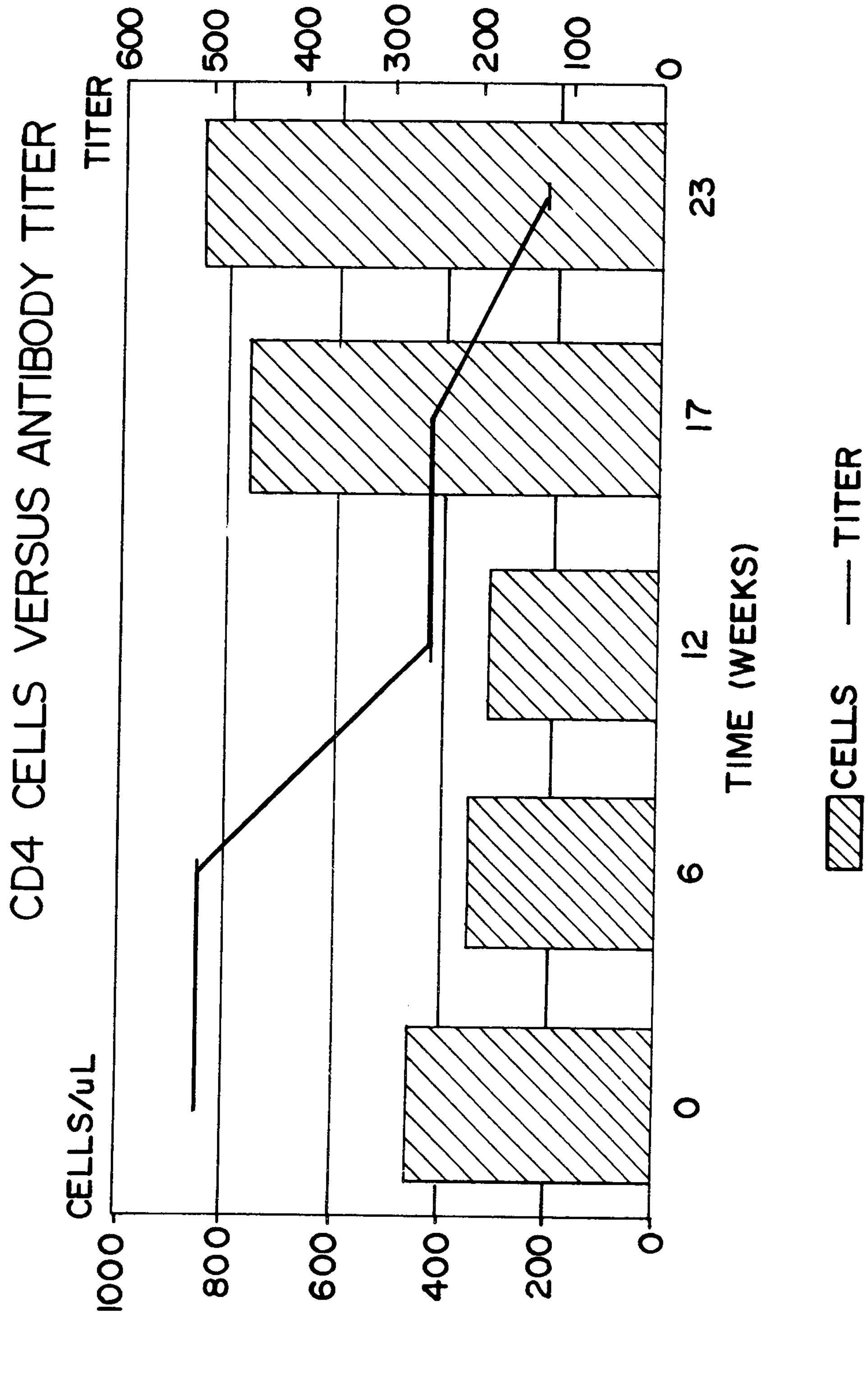
CD4 CELLS VERSUS ANTIBODY TITER
CELLS/uL
TITER
1000
800
600
400
200
0
600
500
400
300
200
100
0
0
6
12
17
23
TIME (WEEKS)
CELLS
TITER

FIG 3